# DEMANDE DE BREVET EUROPEEN

(11) **EP 0 636 686 A1**
(43) Date de publication de la demande: **01.02.1995**
(21) Numéro de dépôt: 94401720.1
(22) Date de dépôt: 27.07.1994
(51) Int. Cl.: C11D 3/22, C11D 1/94, A61K 7/50, A61K 7/06

(54) **Composition de lavage des fibres kératiniques à base de polymères dérivés du chitosane**

(30) Priorité: 28.07.1993 FR 9309299
(71) Demandeur: L'OREAL, F-75008 Paris (FR)
(72) Inventeur: Beauquey, Bernard, F-92110 Clichy (FR); Decoster, Sandrine, F-93800 Epinay-sur-Seine (FR)
(74) Mandataire: Casalonga, Axel

(57) **Abrégé**

L'invention est relative à une composition de lavage contenant dans un milieu aqueux approprié pour le lavage des fibres kératiniques, au moins un agent tensio-actif anionique comportant un ou plusieurs groupements sulfonate, au moins un agent tensio-actif de la famille des bétaïniques et au moins un polymère dérivé du chitosane, le rapport agent tensio-actif anionique/agent tensio-actif bétainique étant supérieur à 0,7.

## Description

La présente invention est relative à une composition destinée au lavage et au traitement des fibres kératiniques et plus particulièrement des cheveux, ainsi qu'aux procédés de lavage mettant en oeuvre une telle composition.

Les polymères dérivés du chitosane sont bien connus dans l'état de la technique et ont déjà été préconisés pour être utilisés dans les compositions de traitement des fibres kératiniques, notamment des cheveux, en vue d'améliorer la tenue dans le temps. De telles compositions sont décrites plus particulièrement dans les brevets français FR-2.486.394 et FR-2.470.596.

De tels polymères ont également été utilisés dans des compositions destinées à lutter contre l'aspect gras des cheveux contenant notamment du lauryléther sulfate de sodium ou du laurylsulfate d'ammonium. Ces compositions ne permettent cependant pas de maintenir correctement la coiffure.

Les compositions de lavage des cheveux ou shampooings sont généralement formulées à base d'agents tensio-actifs anioniques qui sont de bons agents détergents et de bons agents moussants, ou à partir d'agents tensio-actifs amphotères plus doux mais plus onéreux.

Si les agents tensio-actifs anioniques ou amphotères ou leurs associations permettent de nettoyer la chevelure, celle-ci présente généralement une mauvaise tenue au coiffage. Ce problème est habituellement résolu par l'application après le shampooing d'une lotion contenant un polymère susceptible de conférer à la chevelure une tenue au coiffage.

On a également envisagé d'introduire des polymères, en particulier des polymères cationiques, amphotères, anioniques ou leurs mélanges, dans des shampooings à base d'agents tensio-actifs anioniques de la famille des sulfates. On se heurte cependant à des problèmes de solubilisation et lorsque la solubilisation est obtenue, les propriétés de maintien de la coiffure ne sont pas toujours satisfaisantes.

La demanderesse a découvert, ce qui fait l'objet de l'invention, qu'en associant dans un milieu aqueux un agent tensio-actif anionique de la famille des sulfonates, un agent tensio-actif de la famille des bétaïnes et un polymère amphotère dérivé du chitosane, il était possible d'obtenir une composition de lavage homogène, présentant à la fois de bonnes propriétés de détergence et conférant aux cheveux une fois lavés et séchés, une bonne tenue après coiffage.

Un objet de l'invention est donc constitué par des compositions de lavage contenant une telle association.

Un autre objet de l'invention est constitué par un procédé de lavage et de traitement des fibres kératiniques, en particulier les cheveux, mettant en oeuvre la composition précitée.

D'autres objets de l'invention apparaîtront à la lecture de la description et des exemples qui suivent.

La composition de lavage des fibres kératiniques, en particulier des cheveux, conforme à l'invention, est essentiellement caractérisée par le fait qu'elle contient dans un milieu aqueux approprié pour le lavage :
a) au moins un agent tensio-actif anionique comprenant un ou plusieurs groupement(s) sulfonate,
b) au moins un agent tensio-actif bétaïnique,
c) un polymère dérivé du chitosane;

le rapport agent tensio-actif anionique/agent tensio-actif bétaïnique étant égal ou supérieur à 0,7.

De préférence, le rapport agent tensio-actif anionique/bétaïnique est compris entre 0,7 et 4.

La composition conforme à l'invention ne contient pas de polymère cationique et/ou anionique.

Les agents tensio-actifs anioniques de la famille des sulfonates sont choisis plus particulièrement parmi les sels alcalins, alcalino-terreux, d'amines, d'ammonium, d'amino-alcools, des α-oléfine sulfonates, des alkylsulfo-succinates, des alkyléther sulfosuccinates, des alkylamide sulfo-succinates, éventuellement oxyéthylénés avec 1 à 10 moles d'oxyde d'éthylène dans lesquels le groupement alkyle comprend de préférence de 8 à 18 atomes de carbone, des acyliséthionates, des N-acyltaurines.

Les agents tensio-actifs bétaïniques sont choisis plus particulièrement parmi les alkylbétaines et les alkylsulfobétaïnes ayant 8 à 18 atomes de carbone dans le groupement alkyle, les alkylamidopropylbétaïnes ayant entre 5 et 15 atomes de carbone dans le groupement alkyle.

Les polymères dérivés du chitosane comportent en particulier des motifs répondant aux formules suivantes : 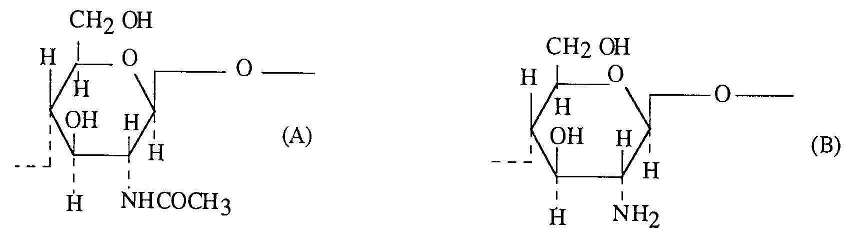 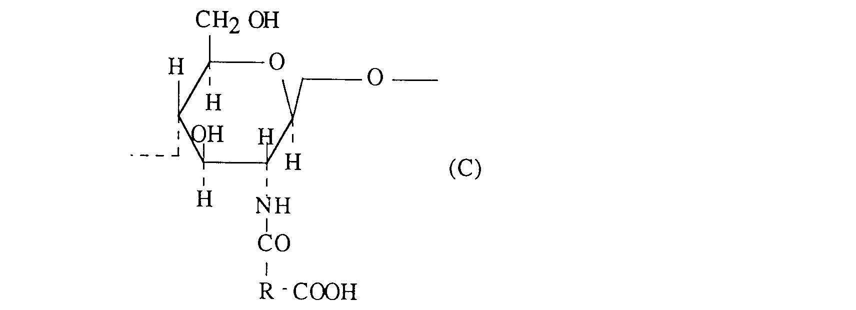
dans lesquelles le motif (A) est présent dans des proportions comprises entre 0 et 30% en poids, le motif (B) est présent dans des proportions comprises entre 5 et 50% en poids et le motif (C) est présent dans des proportions comprises entre 30 et 90% en poids.
Dans la formule (C), R représente un radical de formule : 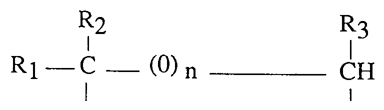
où n désigne zéro ou 1; lorsque n désigne zéro, R₁, R₂, R₃, identiques ou différents, désignent chacun un atome d'hydrogène, un groupement méthyle, hydroxyle, acétoxy, amino, un reste alkylthio dont le groupement alkyle comporte un groupement amino ou un reste monoalkylamine ou dialkylamine, les restes monoalkylamine et dialkylamine pouvant être interrompus par un ou plusieurs atomes d'azote et/ou substitués par un ou plusieurs groupement(s) amine, hydroxyle, carboxyle, alkylthio, sulfonique, l'un au moins des radicaux R₁, R₂ et R₃ étant dans ce cas un atome d'hydrogène; lorsque n désigne 1, chacun des radicaux R₁, R₂ et R₃ représente un atome d'hydrogène.

Alkyle désigne de préférence un groupement ayant 1 à 6 atomes de carbone.

Le polymère constitué des motifs (A), (B) et (C) ou (B) et (C) ci-dessus, peut également se présenter sous forme d'un sel formé avec une base ou avec un acide cosmétiquement acceptable.

Les polymères dérivés du chitosane, plus particulièrement préférés, sont les polymères comportant de 0 à 20% en poids de motif (A), de 40 à 50% en poids de motif (B) et de 40 à 50% en poids de motif (C), dans lequel R désigne un radical CH₂-CH₂-.

Ces polymères sont connus en eux-mêmes et peuvent notamment être préparés par acylation du chitosane avec un anhydride d'acide tel que décrit dans le brevet français FR-2.137.684.

Le milieu aqueux peut être constitué uniquement par de l'eau ou par un mélange d'eau et d'un solvant cosmétiquement acceptable, tel qu'un alcool inférieur en C₁-C₄ comme l'éthanol, l'isopropanol, le n-butanol; les alkylèneglycols comme l'éthylèneglycol, les éthers de glycol. Le pH de ces compositions est généralement compris entre 3 et 9 et plus particulièrement entre 4 et 8.

Les agents tensio-actifs sont utilisés dans les compositions conformes à l'invention dans des proportions suffisantes pour conférer un caractère détergent à la composition et sont compris entre 5 et 50% par rapport au poids total de la composition et en particulier entre 8 et 35%, le rapport entre les agents tensio-actifs anioniques de la famille des sulfonates aux agents tensio-actifs de la famille des bétaïnes est supérieur à 0,7 et de préférence compris entre 0,7 et 4.

Les polymères dérivés du chitosane sont généralement utilisés dans les compositions conformes à l'invention dans des proportions comprises entre 0,1 et 5% en poids et en particulier entre 0,25 et 2% en poids par rapport au poids total de la composition.

Des compositions particulièrement préférées, conformes à l'invention, comprennent :
a) de 4 à 8 % d'alkylsulfosuccinate,
b) de 3 à 7 % d'α-oléfine sulfonate,
c) de 2,5 à 10 % d'une alkylbétaïne,
d) de 0,5 à 5 % de dérivés du chitosane tels que définis ci-dessus,

le rapport alkylsulfosuccinate + α-oléfine sulfonate/alkylbétaïne étant compris entre 0,7 et 4.

Selon une forme de réalisation préférentielle, le rapport des agents tensio-actifs anioniques à groupement sulfonique aux agents tensioactifs bétaïniques est compris entre 1 et 3.

Les compositions de lavage telles que définies ci-dessus peuvent également contenir en plus de l'association définie ci-dessus, des additifs habituellement utilisés dans des shampooings tels que les agents régulateurs de viscosité tels que des électrolytes, des hydrotropes ou d'autres agents épaississants. On peut citer plus particulièrement le chlorure de sodium, le xylène sulfonate de sodium, les scléroglucanes, les gommes de xanthane, les alcanolamides d'acide de coprah, les alcanolamides d'acide alkyléther carboxyliques éventuellement oxyéthylénés avec jusqu'à 5 moles d'oxyde d'éthylène. Ces agents régulateurs de viscosité sont utilisés dans les compositions conformes à l'invention dans des proportions pouvant aller jusqu'à 10% en poids par rapport au poids total de la composition.

Les compositions conformes à l'invention peuvent également contenir jusqu'à 3% d'agents nacrants ou opacifiants bien connus dans l'état de la technique, tels que des palmitates de sodium ou de magnésium, des stéarates ou hydroxystéarates de sodium ou de magnésium, des monostéarates ou distéarates d'éthylèneglycol ou des éthers d'alcool gras comprenant de 27 à 44 atomes de carbone.

Ces compositions peuvent également renfermer d'autres agents ayant pour effet d'améliorer les propriétés cosmétiques des cheveux sans altérer cependant la stabilité des compositions, tels que des agents tensio-actifs cationiques, des polymères non-ioniques ou des protéines. La composition ne contient pas de silicone à groupement hydroxyarylamino, dans une forme de réalisation préférée de l'invention.

Elles peuvent également contenir différents adjuvants utilisés couramment en cosmétique tels que des parfums, des conservateurs, des agents séquestrants, des stabilisateurs de mousse, des agents acidifiants ou alcalinisants bien connus en cosmétique.

Le procédé de lavage des fibres kératiniques et en particulier des cheveux humains, conforme à l'invention, est essentiellement caractérisé par le fait que l'on applique sur des cheveux humides au moins une composition telle que définie ci-dessus et qu'après massage et qu'après un éventuel temps de pose de quelques minutes, on procède au rinçage à l'eau des cheveux. On constate, après séchage et coiffage, que les cheveux présentent une très bonne tenue.

Les exemples suivants sont destinés à illustrer l'invention sans pour autant présenter un caractère limitatif.

### EXEMPLE 1

- Lauryléther sulfosuccinate disodique oxyéthyléné (1 à 4 moles d'oxyde d'éthylène), vendu à 40% de MA sous la dénomination "SETACIN 103 SPECIAL" par la Société ZSCHIMMER & SCHWARZ 6 g MA
- α-oléfine sulfonate, vendu à 38% de MA sous la dénomination "ELFAN OS 46" par la Société AKZO 4,6 g MA
- Cocamidopropylbétaïne vendue à 30% de MA sous la dénomination "TEGO BETAIN HS" par la Société GOLDSCHMIDT 3,6 g MA
- Polymère A 1 g
- HCl qs pH=5,3
- Eau qsp 100 g

Les cheveux traités présentent une tenue améliorée déterminée par le test d'écrasement de boucle décrit ci-après.

Le polymère A répond à la formule suivante comportant les unités B et C dans un rapport 50/50 en poids.

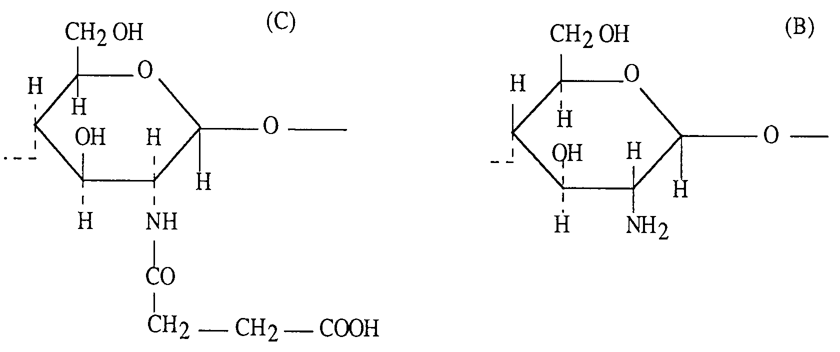

### EXEMPLE 2

- Lauryléther sulfosuccinate disodique oxyéthyléné (1 à 4 moles d'oxyde d'éthylène), vendu à 40% de MA sous la dénomination "SETACIN 103 SPECIAL" par la Société ZSCHIMMER & SCHWARZ 6 g MA
- α-oléfine sulfonate, vendu à 38% de MA sous la dénomination "ELFAN OS 46" par la Société AKZO 4,6 g MA
- Cocamidopropylbétaïne vendue à 30% de MA sous la dénomination "TEGO BETAIN HS" par la Société GOLDSCHMIDT 3,6 g MA
- Polymère A 2 g
- HCl qs pH=5,3
- Eau qsp 100 g

Les cheveux traités présentent une tenue améliorée déterminée par le test d'écrasement de boucle décrit ci-après.

Le polymère A répond à la formule suivante comportant les unités B et C dans un rapport 50/50 en poids.

### EXEMPLE 3

- Lauryléther sulfosuccinate disodique oxyéthyléné (1 à 4 moles d'oxyde d'éthylène), vendu à 40% de MA sous la dénomination "SETACIN 103 SPECIAL" par la Société ZSCHIMMER & SCHWARZ 6 g MA
- α-oléfine sulfonate, vendu à 38% de MA sous la dénomination "ELFAN OS 46" par la Société AKZO 4,6 g MA
- Cocamidopropylbétaïne vendue à 30% de MA sous la dénomination "TEGO BETAIN HS" par la Société GOLDSCHMIDT 10,6 g MA
- Polymère A 2 g
- HCl qs pH=5,3
- Eau qsp 100 g

Les cheveux traités présentent une tenue améliorée déterminée par le test d'écrasement de boucle décrit ci-après.

Le polymère A répond à la formule suivante comportant les unités B et C dans un rapport 50/50 en poids.

### TEST D'ECRASEMENT DE BOUCLE

Les compositions à tester sont appliquées à plat sur 6 mèches de 2,5 g à raison de 0,6 g par mèche. Les mèches rincées et essorées sont enroulées en spires jointives sur un rouleau plein de 20 mm de diamètre, la fixation se faisant par un élastique à chaque extrémité du rouleau.

Les mèches sont séchées sous casque 45 minutes à 60°C. On les laisse ensuite se stabiliser 1 heure à l'ambiante. Puis elles sont dégagées délicatement du rouleau sans modifier leur forme.

La boucle est placée longitudinalement dans un berceau relié à une jauge de contrainte. On abaisse sur la boucle une tige de fer horizontale qui la comprime en la ramenant à une épaisseur constante de 15 mm environ. La jauge affiche la force d'écrasement en grammes. Pour chaque boucle, on effectue 4 mesures en changeant sa position à chaque fois. Pour chaque composition, on effectue les mesures sur 6 mèches.

Les valeurs moyennes obtenues figurent dans le tableau 1 ci-dessus.

| | **Exemple 1** | **Exemple 2** | **Exemple 3** |
|---|---|---|---|
| moyenne de 4 mesures sur 6 mèches | 431 | 460 | 498 |

En effectuant les mêmes mesures avec les compositions décrites dans les exemples 1 ou 2 mais sans le polymère A, on aboutit à une valeur de 256.

## Revendications

1. Composition de lavage, caractérisée par le fait qu'elle contient dans un milieu aqueux approprié pour le lavage des fibres kératiniques, au moins un agent tensio-actif anionique comportant un ou plusieurs groupements sulfonate, au moins un agent tensio-actif de la famille des bétaïniques et au moins un polymère dérivé du chitosane, le rapport agent tensio-actif anionique/agent tensio-actif bétaïnique étant supérieur à 0,7.

2. Composition selon la revendication 1, caractérisée par le fait que le rapport agent tensio-actif anionique/agent tensio-actif bétainique est compris entre 0,7 et 4 en poids.

3. Composition selon l'une quelconque des revendications 1 ou 2, caractérisée par le fait que les agents tensio-actifs anioniques comportant des groupements sulfonates sont choisis parmi les sels alcalins, alcalino-terreux, d'amines, d'ammonium, d'amino-alcools, des α-oléfine sulfonates, des alkylsulfosuccinates, des alkyléther sulfosuccinates, des alkylamide sulfosuccinates éventuellement oxyéthylénés avec 1 à 10 moles d'oxyde d'éthylène dans lesquels le groupement alkyle comprend de préférence de 8 à 18 atomes de carbone, des acyliséthionates, des N-acyltaurines.

4. Composition selon l'une quelconque des revendications 1 ou 2, caractérisée par le fait que les agents tensio-actifs bétaïniques sont choisis parmi les alkylbétaïnes et les alkylsulfobétaïnes ayant un nombre d'atomes de carbone compris entre 8 et 18, les alkylamidopropylbétaïnes ayant un nombre d'atomes de carbone compris entre 5 et 15.

5. Composition selon l'une quelconque des revendications 1 à 4, caractérisée par le fait que le polymère dérivé du chitosane comporte des motifs répondant aux formules : 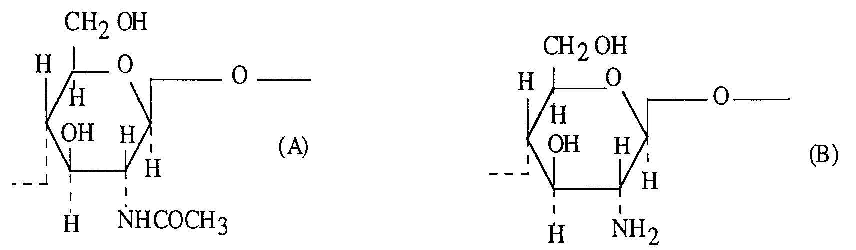 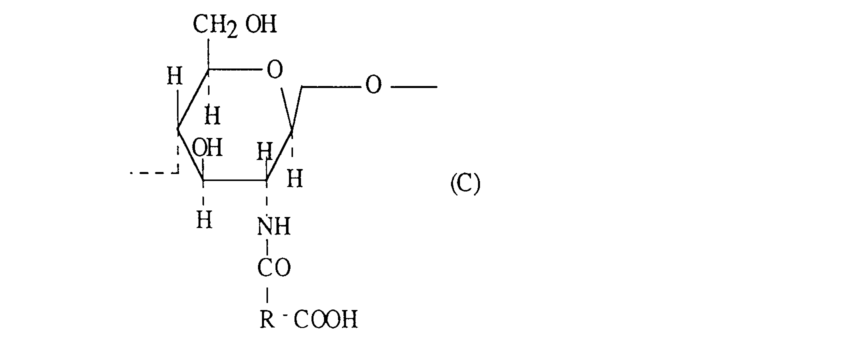 dans lesquelles le motif (A) est présent dans des proportions comprises entre 0 et 30% en poids, le motif (B) est présent dans des proportions comprises entre 5 et 50% en poids et le motif (C) est présent dans des proportions comprises entre 30 et 90% en poids; dans la formule (C), R représente un radical de formule : 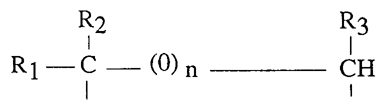 où n désigne zéro ou 1; lorsque n désigne zéro, R₁, R₂, R₃, identiques ou différents, désignent chacun un atome d'hydrogène, un groupement méthyle, hydroxyle, acétoxy, amino, un reste alkylthio dont le groupement alkyle comporte un groupement amino ou un reste monoalkylamine ou dialkylamine; les restes monoalkylamine ou dialkylamine pouvant être interrompus par un ou plusieurs atomes d'azote et/ou substitués par un ou plusieurs groupement(s) amine hydroxyle, carboxyle, alkylthio, sulfonique, l'un au moins des radicaux R₁, R₂ et R₃ étant dans ce cas un atome d'hydrogène; lorsque n désigne 1, chacun de R₁, R₂ et R₃ représente un atome d'hydrogène, ces polymères pouvant se présenter sous forme d'un sel formé avec une base ou un acide cosmétiquement acceptable.

6. Composition selon la revendication 5, caractérisée par le fait que le polymère dérivé du chitosane comporte de 0 à 20% en poids de motif (A), de 40 à 50% en poids de motif (B), de 40 à 50% en poids de motif (C) dans lequel R désigne le radical -CH₂-CH₂-.

7. Composition selon l'une quelconque des revendications 1 à 6, caractérisée par le fait qu'elle comprend :
de 4 à 8 % d'alkylsulfosuccinate,
de 3 à 7 % d'oc-oléfine sulfonate,
de 2,5 à 10 % d'une alkylbétaïne,
de 0,5 à 5 % de dérivés du chitosane.

8. Procédé de lavage des fibres kératiniques, caractérisé par le fait que l'on applique sur des fibres mouillées au moins une des compositions telles que définies dans l'une quelconque des revendications 1 à 7 et qu'on procède au rinçage à l'eau.
